# EUROPEAN PATENT APPLICATION

(11) **EP 4 635 985 A1**
(43) Date of publication of application: **22.10.2025**
(21) Application number: 23904066.0
(22) Date of filing: 15.12.2023
(51) Int. Cl.: C07K 16/30, G01N 33/574, A61P 35/00, A61K 39/00

(54) **ANTI-TM4SF4 HUMANIZED ANTIBODIES AND USE THEREOF**

(30) Priority: 16.12.2022 KR 20220177553
(71) Applicant: Korea Atomic Energy Research Institute, Daejeon 34057 (KR)
(72) Inventor: KIM, In Gyu, Daejeon 34140 (KR); KIM, Rae Kwon, Seoul 05041 (KR); KAHM, Yeon Jee, Hwaseong-si, Gyeonggi-do 18452 (KR); SHIN, Byung Chul, Seoul 04355 (KR); KIM, Sung Chul, Jinju-si, Gyeongsangnam-do 52694 (KR); KIM, Bum Jin, Gwangju 61957 (KR)
(74) Representative: Cabinet Beau de Loménie
(86) International application number: PCT/KR2023/020784
(87) International publication number: WO 2024/128867

(57) **Abstract**

The present invention relates to: humanized antibodies capable of specifically binding to TransMembrane 4 Superfamily Member 4 (TM4SF4) with high affinity and showing low immunogenicity in humans; and a composition for preventing and treating cancer or assisting in immune anticancer therapy, the composition comprising the humanized antibodies.

## Description

### TECHNICAL FIELD

The present invention relates to a humanized antibody capable of specifically binding to TransMembrane 4 Superfamily Member 4 (TM4SF4) with high affinity and exhibiting low immunogenicity in a human, and a composition containing the same for preventing or treating cancer or assisting cancer treatment.

### BACKGROUND ART

### [CROSS-REFERENCE TO RELATED APPLICATIONS]

This application claims priority from Korean Patent Application No. 10-2022-0177553, filed on December 16, 2022, the disclosure of which is incorporated herein by reference in its entirety.

TransMembrane 4 Superfamily Member 4 (TM4SF4) is a type of tetraspanin protein, other proteins in this class, such as TM4SF1 and TM4SF5, are reported to have expression upregulated in many types of tumors and involved in epithelial-mesenchymal transition and cell migration, and many studies related to cancer cells have been conducted. TM4SF4 has been reported to be involved in apoptosis, differentiation, and cell invasion ability in cancer cells in some reports, and recently, the research team of the present invention reported that TM4SF4 protein promotes cancer stem cell growth, self-renewal ability, and metastasis/invasion in human lung cancer cells, and suggested that TM4SF4 promotes the activation of IGF1Rβ/AKT/NFκB or JAK2 (or FAK)/STAT3, which is an important signaling system for cancer occurrence, which enhances cancer stem cell characteristics through the promoted cytokine secretion, thereby making tumors more malignant (Choi SI et al., Oncotarget. 2014; 5 (20):9823-9837, Choi SI et al., Oncotarget. 2017; 8 (60):101284-101297).

An antibody has been utilized as a therapeutic agent because of their high binding specificity for a target antigen and stability in a human body. In particular, antibodies having anticancer functions have been utilized to greatly enhance the efficacy of cancer treatment after being improved into humanized antibodies, single-chain antibodies, bispecific antibodies, and drug-fusion antibodies based on the development of antibody engineering technology. However, due to the diversity of cancer characteristics and the induction of treatment resistance by the expression of new antigens, limitations have been pointed out in the types of existing antigens that have been used to target cancer cells, and studies to search for a novel cancer-specific antigen and derive an antibody against the same have been continuously conducted.

In particular, in the case of cancer that is resistant to a target drug or radiotherapy used in existing cancer treatments and relapses, it has been reported that the characteristics of cancer stem cells play an important role, and discovery of antigens that may be used to target cancer stem cells and securing specific antibodies are becoming increasingly important.

Meanwhile, in order to develop a monoclonal antibody that specifically binds to a specific antigen, a method of injecting an antigen into an animal other than a human and utilizing an antibody produced by the immune system of the animal has been mainly used. However, the antibody produced through the above method is not a human protein in that it is derived from an animal other than a human, and therefore, problems with immunogenicity may occur when administered to a human. That is, when an antibody derived from a species other than a human is administered to a human body, it may induce the generation of human anti-mouse antibody (HAMA), and the therapeutic efficacy of the heterologous antibody may be reduced.

Accordingly, a method is proposed to solve the problems described above by obtaining a sequence that plays an important role in binding to an antigen among the amino acid sequences constituting the antibody derived from a species other than a human, and replacing the remaining part with a sequence of a human antibody. However, there is a possibility that a chimeric antibody or a humanized antibody produced through the method may not function as an antibody or its binding affinity for an antigen may be reduced because it is a fusion of two different protein regions. Therefore, it is still difficult to develop a humanized antibody that shows high specificity and affinity for an antigen without inducing the generation of HAMA in a human body.

In addition, a need for a method to maximize the effectiveness of anticancer immunotherapy by inhibiting TM4SF4 expressed in cancer cells and increasing the activity of anticancer immunity has emerged. Accordingly, the inventors of the present invention have tried to develop an antibody that assists anticancer immunotherapy by inhibiting activity of TM4SF4 protein of cancer cells.

### DISCLOSURE OF THE INVENTION

### TECHNICAL PROBLEM

An object of the present invention is to provide a novel humanized antibody or antigen-binding fragment thereof that may specifically bind, with high affinity, to TransMembrane 4 Superfamily Member 4 (M4SF4) protein that is overexpressed on a surface of a cancer cell, and may exhibit low immunogenicity when administered to a human body.

Another object of the present invention is to provide a polynucleotide encoding the humanized antibody or antigen-binding fragment thereof and capable of expressing the same, an expression vector, a host cell, and a method for producing the humanized antibody or antigen-binding fragment thereof. Still another object of the present invention is to provide a method for producing an antibody or antigen-binding fragment thereof, the method including culturing the host cell.

Still another object of the present invention is to provide a composition and a kit for detecting TM4SF4, and a method for detecting TM4SF4.

Still another object of the present invention is to provide a pharmaceutical composition for preventing or treating cancer, a composition for inhibiting cancer stem cell growth, and a composition for assisting radiation anticancer treatment.

Still another object of the present invention is to provide a composition for assisting anticancer immunotherapy.

### TECHNICAL SOLUTION

One aspect of the present invention provides a humanized antibody or antigen-binding fragment thereof containing: a heavy chain variable region containing FR-H1 having an amino acid sequence of SEQ ID NO: 1, FR-H2 having an amino acid sequence of SEQ ID NO: 2, FR-H3 having an amino acid sequence of SEQ ID NO: 3, and FR-H4 having an amino acid sequence of SEQ ID NO: 4; and a light chain variable region containing FR-L1 having an amino acid sequence of SEQ ID NO: 5, FR-L2 having an amino acid sequence of SEQ ID NO: 6, FR-L3 having an amino acid sequence of SEQ ID NO: 7, and FR-L4 having an amino acid sequence of SEQ ID NO: 8, wherein the humanized antibody or antigen-binding fragment thereof specifically binds to TransMembrane 4 Superfamily Member 4 (TM4SF4).

In addition, in the humanized antibody or antigen-binding fragment thereof, the heavy chain variable region further contains at least one CDR selected from the group consisting of CDR-H1 having an amino acid sequence of SEQ ID NO: 9, CDR-H2 having an amino acid sequence of SEQ ID NO: 10, and CDR-H3 having an amino acid sequence of SEQ ID NO: 11, and the light chain variable region further contains at least one CDR selected from the group consisting of CDR-L1 having an amino acid sequence of SEQ ID NO: 12 or SEQ ID NO: 13, CDR-L2 having an amino acid sequence of SEQ ID NO: 14, and CDR-L3 having an amino acid sequence of SEQ ID NO: 15.

In another aspect of the present invention, in the humanized antibody or antigen-binding fragment thereof, the humanized antibody or antigen-binding fragment thereof has an amino acid sequence in which asparagine, which is an amino acid, is substituted with phenylalanine at position 31 of CDR1 in the light chain variable region.

In still another aspect of the present invention, there are provided a polynucleotide having a base sequence encoding the two types of humanized antibody or antigen-binding fragment, an expression vector containing the polynucleotide, and a host cell containing the expression vector.

Still another aspect of the present invention provides a composition for detecting TM4SF4 containing the humanized antibody or antigen-binding fragment, and a kit for detecting TM4SF4 containing the composition for detecting TM4SF4.

Still another aspect of the present invention provides a method for detecting TM4SF4, the method including bringing the humanized antibody or antigen-binding fragment thereof into contact with a sample to be detected that is expected to contain TM4SF4.

Still another aspect of the present invention provides a pharmaceutical composition for preventing or treating cancer, a composition for inhibiting cancer stem cell growth, and a composition for assisting radiation anticancer treatment, containing the humanized antibody or antigen-binding fragment thereof.

### ADVANTAGEOUS EFFECTS

The humanized antibody of the present invention may be usefully used to detect TM4SF4 or target cancer cells that overexpress TM4SF4 or cancer stem cells since it may specifically bind to TM4SF4 without binding to substances such as BSA. In particular, the humanized antibody of the present invention exhibits excellent effects because it has a significantly higher binding affinity compared to an antibody derived from a mouse or a chimeric antibody.

In addition, since the humanized antibody of the present invention uses an amino acid sequence derived from a human antibody or is obtained by modifying a part of the amino acid sequence except for a CDR sequence, the humanized antibody of the present invention is unlikely to induce human anti-mouse antibody (HAMA) when administered to a human body, and thus has the effect of low immunogenecity. Accordingly, the humanized antibody of the present invention has the advantage of being able to solve problems of immune response that may occur when using an antibody derived from a mouse.

In addition, the humanized antibody of the present invention may reduce the growth, migration and invasion, and radiation resistance of cancer cells, and may lower the radiation resistance of cancer cells, thereby preventing or treating cancer, inhibiting cancer stem cell growth, and assisting radiation anticancer treatment.

In addition, the humanized antibody of the present invention has an excellent effect on immune anticancer by inhibiting the expression of PD-L1 and enhancing antibody-dependent cellular cytotoxicity.

However, the effects of the present invention are not limited to the effects mentioned above, and other effects that are not mentioned may be obviously understood by those skilled in the art from the following description.

### BRIEF DESCRIPTION OF THE DRAWINGS

FIG. 1 illustrates the results of a colony formation assay to confirm changes in cell growth according to treatment of lung adenocarcinoma cell line A549 and pancreatic cancer cell line MIAPaCa-2 that express TM4SF4, and H1299 cell line that does not express TM4SF4 with an anti-TM4SF4 humanized antibody.
FIG. 2 illustrates a result of confirming changes in radiation resistance through a colony formation assay when the humanized antibody of the present invention is treated in lung adenocarcinoma cell line A549, and MIAPaCa-2 cells, which are a pancreatic cancer cell line.
FIG. 3 illustrates a result of confirming changes in cell migration through wound healing when the humanized antibody of the present invention is treated in lung adenocarcinoma cell line A549, and MIAPaCa-2 cells, which are a pancreatic cancer cell line.
FIG. 4 illustrates a result of confirming changes in cell migration and invasion through a transwell when the humanized antibody of the present invention is treated in lung adenocarcinoma cell line A549, and MIAPaCa-2 cells, which are a pancreatic cancer cell line.
FIG. 5 illustrates a result of confirming changes in stemness of cancer cells through cell sphere formation when the humanized antibody of the present invention is treated in lung adenocarcinoma cell line A549, and MIAPaCa-2 cells, which are a pancreatic cancer cell line.
FIG. 6 illustrates a result of confirming through Western blotting that EMT phenomenon of cells is significantly inhibited when the humanized antibody of the present invention is treated in lung adenocarcinoma cell line A549, and MIAPaCa-2 cells, which are a pancreatic cancer cell line.
FIG. 7 illustrates a result of confirming through immunofluorescence that EMT phenomenon of cells is significantly inhibited when the humanized antibody of the present invention is treated in lung adenocarcinoma cell line A549, and MIAPaCa-2 cells, which are a pancreatic cancer cell line.
FIG. 8 illustrates a result of confirming the expression levels of ALDH1 and CD44 within cells when the humanized antibody of the present invention is treated in lung adenocarcinoma cell line A549, and MIAPaCa-2 cells, which are a pancreatic cancer cell line.
FIG. 9 illustrates a result of confirming the antibody-dependent cellular cytotoxicity (ADCC) function of the humanized antibody of the present invention in lung adenocarcinoma cell line A549, and MIAPaCa-2 cells, which are a pancreatic cancer cell line.
FIG. 10 illustrates a result of confirming changes in protein and gene expression of programmed death-ligand 1 (PD-L1) and B7H4, which are ligands of immune checkpoint receptors in cancer cells, when the humanized antibody of the present invention is treated in lung adenocarcinoma cell line A549, and MIAPaCa-2 cells, which are a pancreatic cancer cell line.

### BEST MODE FOR CARRYING OUT THE INVENTION

Hereinafter, the present invention will be described in detail.

### 1. Humanized antibody or antigen-binding fragment thereof that specifically binds to TM4SF4

An aspect of the present invention provides a humanized antibody or antigen-binding fragment thereof that specifically binds to TM4SF4 and exhibits low immunogenicity in a human body.

The term "antibody" in the present invention refers to an immunoglobulin molecule having immunological responsiveness by specifically binding to an epitope of an antigen. The antibody may include all of a monoclonal antibody, a polyclonal antibody, an antibody having a full-length chain structure (a full-length antibody), a functional fragment having at least an antigen-binding function (antigen-binding fragment), and a recombinant antibody, and specifically, the antibody of the present invention may be a monoclonal antibody or an antigen-binding fragment thereof. The monoclonal antibody refers to an antibody molecule of a single molecular composition obtained from a substantially identical antibody population, and the monoclonal antibody exhibits single binding specificity and affinity for a specific epitope. The full-length antibody has a structure having two full-length light chains and two full-length heavy chains, and each light chain may be linked to the heavy chain through a disulfide bond. The antibody may contain heavy chain (HC) and light chain (LC) polypeptides, and the heavy chain and the light chain may include a variable region and a constant region.

The constant region is a site that mediates binding of the antibody to various cells of an immune system (T-cell, and the like), host tissue containing components of a complement system, and the like. The constant region has the same function regardless of the type of antigen as long as it is the same type of antibody derived from the same species, and the amino acid sequence constituting the constant region is identical or highly similar for each antibody. The constant region may be divided into a heavy chain constant region (may be abbreviated as CH) and a light chain constant region (may be abbreviated as CL). The heavy chain constant region has gamma (γ), mu (µ), alpha (α), delta (δ), and/or epsilon (ε) type, and has gamma 1 (γ1), gamma 2 (γ2), gamma 3 (γ3), gamma 4 (γ4), alpha 1 (α1), and/or alpha 2 (α2) as a subclass. The light chain constant region has kappa (κ) and lambda (λ) types. IgG includes IgG1, IgG2, IgG3, and IgG4 as a subtype.

The variable region is an antibody site that has specificity for an antigen, and may be divided into a heavy chain variable region (may be abbreviated as VH) and a light chain variable region (may be abbreviated as VL). The variable region may include three complementary-determining regions (CDRs) and four framework regions (FRs). The CDR may be a ring-shaped site involved in recognition of an antigen, and specificity for the antigen may be determined according to an amino acid sequence of the CDR.

The CDRs may be referred to as CDR1, CDR2, and CDR3 according to the order, and may be referred to as CDR-H1, CDR-H2, and CDR-H3 for the heavy chain variable regions and CDR-L1, CDR-L2, and CDR-L3 for the light chain variable regions, depending on whether the CDR is of the heavy chain or light chain polypeptide.

The FR refers to a part of a variable region of an immunoglobulin molecule other than a complementary-determining region, and the FRs may be referred to as FR1, FR2, FR3, and FR4 according to the order and may be referred to as FR-H1, FR-H2, FR-H3, and FR-H4 for the heavy chain variable regions and FR-L1, FR-L2, FR-L3, and FR-H4 for the light chain variable regions, depending on whether the FR is of the heavy chain or light chain polypeptide.

Similarly, the FRs may be referred to as FR-H1, FR-H2, FR-H3, and FR-H4 for the heavy chain variable regions, and may be referred to as FR-L1, FR-L2, FR-L3, and FR-L4 for the light chain variable regions. In addition, the CDRs and the FRs may be arranged in the following order in each variable region. The order is from N-terminus (amino-terminus) to C-terminus (carboxy-terminus): FR-H1, CDR-H1, FR-H2, CDR-H2, FR-H3, CDR-H3, and FR-H4 for the heavy chain variable region; and FR-L1, CDR-L1, FR-L2, CDR-L2, FR-L3, CDR-L3, and FR-L4 for the light chain variable region.

The term "antigen-binding fragment" in the present invention refers to any fragment of the humanized antibody of the present invention that has the antigen-binding function of the antibody. The antigen-binding fragment may be referred to interchangeably with terms such as "fragment" and "antibody fragment", and the antigen-binding fragment may be Fab, Fab', F(ab')₂, Fv, or the like, but is not limited thereto.

The Fab has a structure having variable regions of a light chain and a heavy chain, a constant region of the light chain, and a first constant region of the heavy chain (CH1 domain), and has one antigen-binding site. The Fab' differs from the Fab in that the Fab' has a hinge region containing one or more cysteine residues at the C-terminus of the heavy chain CH1 domain. The F(ab')₂ is generated when the cysteine residue of the hinge region of the Fab' forms a disulfide bond. The Fv refers to the minimum antibody fragment with only a heavy chain variable region and a light chain variable region. The two-chain Fv may be formed by linking the heavy chain variable region and the light chain variable region through a non-covalent bond, and the single-chain Fv may be generally formed by linking the heavy chain variable region and the light chain variable region via a peptide linker through a covalent bond or directly linking the heavy chain variable region and the light chain variable region at the C-terminus, thereby forming a structure such as a dimer like the two-chain Fv. The antigen-binding fragment may be prepared by using a protease (for example, the Fab may be obtained by restriction cleavage of the entire antibody with papain, and the F(ab')2 fragment may be obtained by cleavage of the entire antibody with pepsin), or through a genetic recombination technology, but is not limited thereto.

The term "humanized antibody" in the present invention refers to an antibody that exhibits reduced immunogenicity or is non-immunogenic in a human. The humanized antibody may be produced by combining, for example, a CDR derived from a non-human entity (non-human species) with a constant region derived from a human antibody and an FR among variable regions derived from a human antibody. The humanized antibody may be produced by grafting a CDR of a non-human species antibody between FR sequences of a human antibody through a CDR-grafting method.

The humanized antibody refers to an antibody in which both heavy chain and light chain variable regions are derived from a human. Depending on a difference in heavy chain constant region, a human antibody contains IgG having a γ-chain heavy chain (including IgG1, IgG2, IgG3, and IgG4), IgM having a µ-chain heavy chain, IgA having an α-chain heavy chain (including IgA1 and IgA2), IgD having a δ-chain heavy chain, or IgE having an ε-chain heavy chain. In addition, in principle, the light chain contains at least one of a κ-chain and a λ-chain.

The humanized antibody has the characteristic of having a higher similarity to a human antibody compared to a mouse antibody or a chimeric antibody, and thus having lower immunogenicity when administered to a human.

The term "epitope" in the present invention refers to a specific site on an antigen that may be specifically recognized and bound by an immunoglobulin, an antibody, or an antigen-binding fragment thereof. The epitope may be formed from contiguous amino acids or from noncontiguous amino acids juxtaposed by a tertiary folding of a protein.

The TM4SF4 is a type of tetraspanin protein and is a protein known to be involved in apoptosis, differentiation, and cell invasion ability in cancer cells. In addition, the TM4SF4 is known to promote the growth and metastasis of cancer stem cells, and may enhance the characteristics of cancer stem cells and make tumor more malignant. The TM4SF4 protein may be a membrane protein existing in a cell membrane, and a portion of the TM4SF4 may be exposed to the outside of the cell. The exposed portion may have two loop structures, and some amino acid sequences of the extracellular exposed portion of the TM4SF4 may be an epitope to which the humanized antibody of the present invention or the antigen-binding fragment thereof may specifically recognize and bind. The epitope of the TM4SF4 may have, for example, an amino acid sequence of "TWGYPFHDGDYLNDE" (in the order from the N-terminus to the C-terminus, SEQ ID NO: 2).

The epitope region may be, for example, GGCARCLGGTLIPLAFFGFLANILLFFPGG (SEQ ID NO: 23) at positions 4 to 33, LGSGVLMIFPALVFL (SEQ ID NO: 24) at positions 53 to 67, NNDCCGCCGN (SEQ ID NO: 25) at positions 71 to 80, STIFAVVGFLGAGYSFIISAI (SEQ ID NO: 26) at positions 92 to 112, KGPKCLM (SEQ ID NO: 27) at positions 116 to 122, WGYPFHD (SEQ ID NO: 28) at positions 127 to 133, and CREPLNVVPWNLTLFSILLVVGGIQMVLCAIQVVNGLLGTLCGDCQCCGCCGG (SEQ ID NO: 29) at positions 146 to 198, from the N-terminus of the reference TM4SF4 antigen (SEQ ID NO: 1), and more specifically, may be TWGYPFHDGDYLNDE (SEQ ID NO: 2) at positions 126 to 140 from the N-terminus in the reference TM4SF4 antigen (SEQ ID NO: 1).

The humanized antibody of the present invention or the antigen-binding fragment thereof contains a heavy chain variable region containing FR-H1 having an amino acid sequence of SEQ ID NO: 1, FR-H2 having an amino acid sequence of SEQ ID NO: 2, FR-H3 having an amino acid sequence of SEQ ID NO: 3, and FR-H4 having an amino acid sequence of SEQ ID NO: 4, and a light chain variable region containing FR-L1 having an amino acid sequence of SEQ ID NO: 5, FR-L2 having an amino acid sequence of SEQ ID NO: 6, FR-L3 having an amino acid sequence of SEQ ID NO: 7, and FR-L4 having an amino acid sequence of SEQ ID NO: 8, and specifically binds to TransMembrane 4 Superfamily Member 4 (TM4SF4).

FR-H1 having the amino acid sequence of SEQ ID NO: 1 may be an FR1 sequence of a heavy chain variable region referred to as "Hz2B7-1.1" or "Hz2B7-1.2" in the present invention. FR-H2 having the amino acid sequence of SEQ ID NO: 2 may be an FR2 sequence of a heavy chain variable region referred to as "Hz2B7-1.1" or "Hz2B7-1.2" in the present invention, and FR-H3 having the amino acid sequence of SEQ ID NO: 3 may be an FR3 sequence of a heavy chain variable region referred to as "Hz2B7-1.1" or "Hz2B7-1.2" in the present invention. FR-H4 having the amino acid sequence of SEQ ID NO: 4 may be an FR4 sequence of a heavy chain variable region referred to as "Hz2B7-1.1" or "Hz2B7-1.2" in the present invention.

FR-L1 having the amino acid sequence of SEQ ID NO: 5 may be an FR1 sequence of a light chain variable region referred to as "Hz2B7-1.1" or "Hz2B7-1.2" in the present invention. FR-L2 having the amino acid sequence of SEQ ID NO: 6 may be an FR2 sequence of a light chain variable region referred to as "Hz2B7-1.1" or "Hz2B7-1.2" in the present invention. FR-L3 having the amino acid sequence of SEQ ID NO: 7 may be an FR3 sequence of a light chain variable region referred to as "Hz2B7-1.1" or "Hz2B7-1.2" in the present invention. FR-L4 having the amino acid sequence of SEQ ID NO: 8 may be an FR4 sequence of a light chain variable region referred to as "Hz2B7-1.1" or "Hz2B7-1.2" in the present invention.

The heavy chain variable region may further contain at least one CDR selected from the group consisting of CDR-H1 having an amino acid sequence of SEQ ID NO: 9, CDR-H2 having an amino acid sequence of SEQ ID NO: 10, and CDR-H3 having an amino acid sequence of SEQ ID NO: 11, and preferably contains the CDR-H1, CDR-H2, and CDR-H3.

The light chain variable region may further contain at least one CDR selected from the group consisting of CDR-L1 having an amino acid sequence of SEQ ID NO: 12 or SEQ ID NO: 13, CDR-L2 having an amino acid sequence of SEQ ID NO: 14, and CDR-L3 having an amino acid sequence of SEQ ID NO: 15, and preferably contains the CDR-L1, CDR-L2, and CDR-L3.

CDR-H1 having the amino acid sequence of SEQ ID NO: 9 may be a CDR1 sequence of a heavy chain variable region referred to as "Hz2B7-1.1" or "Hz2B7-1.2" in the present invention. CDR-H2 having the amino acid sequence of SEQ ID NO: 10 may be a CDR2 sequence of a heavy chain variable region referred to as "Hz2B7-1.1" or "Hz2B7-1.2" in the present invention, and CDR-H3 having the amino acid sequence of SEQ ID NO: 11 may be a CDR3 sequence of a heavy chain variable region referred to as "Hz2B7-1.1" or "Hz2B7-1.2" in the present invention.

CDR-L1 having the amino acid sequence of SEQ ID NO: 12 may be a CDR1 sequence of a light chain variable region referred to as "Hz2B7-1.1" in the present invention, CDR-L1 having the amino acid sequence of SEQ ID NO: 13 may be a CDR1 sequence of a light chain variable region referred to as "Hz2B7-1.2" in the present invention, and CDR-L2 having the amino acid sequence of SEQ ID NO: 14 may be a CDR2 sequence of a light chain variable region referred to as "Hz2B7-1.1" or "Hz2B7-1.2" in the present invention. CDR-L3 having the amino acid sequence of SEQ ID NO: 15 may be a CDR3 sequence of a light chain variable region referred to as "Hz2B7-1.1" or "Hz2B7-1.2" in the present invention.

The heavy chain variable region may have an amino acid sequence of SEQ ID NO: 16.

The light chain variable region may have an amino acid sequence of SEQ ID NO: 17 or SEQ ID NO: 18.

The heavy chain variable region having the amino acid sequence of SEQ ID NO: 16 may be a sequence of a heavy chain variable region referred to as "Hz2B7-1.1" or "Hz2B7-1.2" in the present invention.

The light chain variable region having the amino acid sequence of SEQ ID NO: 17 may be a sequence of a light chain variable region referred to as "Hz2B7-1.1" in the present invention, and the light chain variable region having the amino acid sequence of SEQ ID NO: 18 may be a sequence of a light chain variable region referred to as "Hz2B7-1.2" in the present invention. The humanized antibody of the present invention or the antigen-binding fragment thereof may contain a heavy chain constant region and/or a light chain constant region of an antibody derived from a human, and the heavy chain constant region and/or light chain constant region of the antibody derived from a human may be used without limitation as long as the humanized antibody or antigen-binding fragment thereof does not inhibit the property of specifically binding to TM4SF4.

The amino acid sequences described above may include variants having different sequences due to deletion, insertion, or substitution of an amino acid residue, or a combination thereof within a range that does not affect a structure, function, activity, and the like of the polypeptide containing the same. In addition, the amino acid sequences may contain amino acids that have undergone common modifications known in the art, and examples of the modification of the amino acid include phosphorylation, sulfation, acrylation, glycosylation, methylation, and farnesylation.

In another aspect of the present invention, the humanized antibody or antigen-binding fragment thereof may have an amino acid sequence in which the amino acid asparagine is substituted with phenylalanine at position 31 of CDR1 in the light chain variable region.

The humanized antibody of the present invention or the antigen-binding fragment thereof not only has the amino acid sequences described above, but also has an amino acid sequence that is substantially identical thereto or a variant thereof. The meaning of having the substantially identical amino acid sequence is that an amino acid sequence has 90% or more, 91% or more, 92% or more, 93% or more, 94% or more, 95% or more, 96% or more, 97% or more, 98% or more, 99% or more, or 99.5% or more homology with the amino acid sequence described above, but is not limited thereto.

### 2. Technique for expressing TM4SF4-specific humanized antibody

Still another aspect of the present invention provides a polynucleotide, an expression vector, a host cell, and a production method that can be used to express and produce the humanized antibody or antigen-binding fragment thereof.

Since the descriptions of the humanized antibody, antigen-binding fragment thereof, TM4SF4, and the like are the same as those described in "1. Humanized antibody or antigen-binding fragment thereof that specifically binds to TM4SF4", the descriptions are omitted to avoid repetitive descriptions, and only the contents related to the polynucleotide, expression vector, and host cell are described below.

The term "polynucleotide" in the present invention comprehensively includes DNA and RNA molecules, and a nucleotide, which is the basic structural unit of the polynucleotide, may include not only a nucleotide existing in nature, but also an analogue in which a sugar or base portion is modified.

The polynucleotide of the present invention has a base sequence encoding the humanized antibody or antigen-binding fragment thereof.

Encoding the humanized antibody or antigen-binding fragment thereof means that genetic information that enables a protein having the amino acid sequence of the humanized antibody of the present invention or the antigen-binding fragment thereof to be synthesized is encoded through a normal protein expression process of the polynucleotide, such as transcription or translation. In this case, the scope of the present invention may include even a polynucleotide that encodes not only a protein having an amino acid sequence completely identical to that of the humanized antibody or antigen-binding fragment thereof, but also a protein having an amino acid sequence substantially identical to that of the protein as described above or a protein having the identical and/or similar activity to that of the protein.

The polynucleotide may have an optimized base sequence depending on the type of organism into which the polynucleotide is to be introduced and expressed and the expression system of the organism, such as transcription or translation.

Specifically, the polynucleotide may have a base sequence of SEQ ID NO: 19 or SEQ ID NO: 20.

The base sequence of SEQ ID NO: 19 may encode the amino acid sequence of "Hz2B7-1.1", and the base sequence of SEQ ID NO: 20 may encode the amino acid sequence of "Hz2B7-1.2".

The polynucleotide of the present invention may have a base sequence substantially identical to the base sequences listed above. The substantially identical base sequence includes, for example, a case in which the same amino acid may be synthesized when transcribed and translated, and may be a base sequence having 90% or more, 91% or more, 92% or more, 93% or more, 94% or more, 95% or more, 96% or more, 97% or more, 98% or more, 99% or more, or 99.5% or more homology with the base sequences listed above, but is not limited thereto.

The expression vector of the present invention contains the polynucleotide.

The term "expression vector" in the present invention refers to a means for expressing a specific gene in a host cell, and specifically, the expression vector includes a plasmid vector; a cosmid vector; and a viral vector such as a bacteriophage vector, an adenovirus vector, a retrovirus vector, or an adeno-associated virus vector, but is not limited thereto.

In addition to the base sequence encoding the humanized antibody or antigen-binding fragment thereof, the expression vector may further have a regulatory sequence such as a promoter or a terminator, and the base sequence encoding the humanized antibody or antigen-binding fragment thereof may be operatively linked to a promoter. The operably linked means a functional linkage between a regulatory sequence (for example, a promoter, a signal sequence, an array of a transcriptional regulator binding site, or the like) and another base sequence, whereby the regulatory sequence may regulate transcription and/or translation of the other base sequence.

The expression vector system of the present invention may be constructed through various methods known in the art.

The expression vector may be constructed using a prokaryotic cell or a eukaryotic cell as a host.

For example, when the expression vector uses a prokaryotic cell as a host, the expression vector generally contains a strong promoter capable of advancing transcription (for example, tac promoter, lac promoter, lacUV5 promoter, lpp promoter, pLλ promoter, pRλ promoter, rac5 promoter, amp promoter, recA promoter, SP6 promoter, trp promoter, T7 promoter, or the like), a ribosome binding site for initiating translation, and a transcription/translation termination sequence. When *E. coli* (for example, HB101, BL21, DH5α, or the like) is used as a host cell, a promoter and operator site of an *E. coli* tryptophan biosynthesis pathway (Yanofsky, C, J Bacteriol, (1984) 158:1018-1024) and a leftward promoter of phage λ (pLλ promoter; Herskowitz, I and Hagen, D, Ann Rev Genet, (1980) 14:399-445) may be used as regulatory sites. When *Bacillus* bacteria are used as a host cell, a promoter of toxin protein gene of *Bacillus thuringiensis* (Appl Environ Microbiol (1998) 64:3932-3938; Mol Gen Genet(1996) 250:734-741) or any promoter that may be expressed in *Bacillus* bacteria may be used as a regulatory site. The expression vector may be produced by manipulating a plasmid (for example, pCL, pSC101, pGV1106, pACYC177, ColE1, pKT230, pME290, pBR322, pUC8/9, pUC6, pBD9, pHC79, pIJ61, pLAFR1, pHV14, pGEX series, pET series, pUC19, or the like), a phage (for example, λgt4·λB, λ-Charon, λΔz1, M13, or the like), or a virus (for example, SV40 or the like), which is often used in the art.

In a case where the expression vector uses a eukaryotic cell as a host, a promoter derived from a genome of a mammalian cell (for example, metallothionine promoter, β-actin promoter, human hemoglobin promoter, or human muscle creatine promoter) or a promoter derived from a mammalian virus (for example, adenovirus late promoter, vaccinia virus 75K promoter, SV40 promoter, cytomegalovirus (CMV) promoter, tk promoter of HSV, mouse mammary tumor virus (MMTV) promoter, LTR promoter of HIV, promoter of Moloney virus, promoter of Epstein-Barr virus (EBV), and promoter of Roux Sarcoma virus (RSV)) may be used, and the expression vector may generally have a polyadenylation sequence as a transcription termination sequence. The expression vector may contain a CMV promoter.

In addition, the expression vector may be fused with other sequences to facilitate purification of the antibody expressed therefrom. Examples of the sequences to be fused include glutathione S-transferase (Pharmacia, USA), maltose binding protein (NEB, USA), FLAG (IBI, USA), and 6x His (hexahistidine; Quiagen, USA). In addition, since the protein expressed by the expression vector of the present invention is a humanized antibody or antigen-binding fragment thereof, the expressed protein may be easily purified through a protein A column or the like without an additional sequence for purification in consideration of characteristics thereof.

The expression vector contains an antibiotic resistance gene conventionally used in the art as a selectable marker, and may contain, for example, resistance genes against ampicillin, gentamicin, carbenicillin, chloramphenicol, streptomycin, kanamycin, geneticin, neomycin, and tetracycline.

The expression vector may be a vector system in which a light chain and a heavy chain are simultaneously expressed in one vector, or a system in which a light chain and a heavy chain are expressed in separate vectors, respectively. In the latter case, the two vectors may be introduced into a host cell through, for example, co-transformation or targeted transformation. Co-transformation is a method of simultaneously introducing each vector DNA encoding a light chain and a heavy chain into a host cell and then selecting cells that express both the light chain and the heavy chain. Targeted transformation is a method of selecting cells transformed with a vector containing a light chain (or a heavy chain), transforming the selected cells again with a vector containing a heavy chain (or a light chain), and finally selecting cells that express both the light chain and the heavy chain.

The host cell of the present invention contains the expression vector.

As the host cell, any host cell known in the art may be used as long as it may stably and continuously clone and express the expression vector of the present invention, and examples of the host cell include, but are not limited to, prokaryotic host cells, such as *Escherichia coli, Bacillus* strains such as *Bacillus subtilis* and *Bacillus thuringiensis, Streptomyces, Pseudomonas* such as *Pseudomonas putida, Proteus mirabilis,* and *Staphylococcus* such as *Staphylococcus carnosus.*

When the host cell is a eukaryotic host cell, fungi such as *Aspergillus* species, yeasts such as *Pichia pastoris, Saccharomyces cerevisiae, Schizosaccharomyces,* and *Neurospora crassa,* other lower eukaryotes, higher eukaryotes such as insect-derived cells, and cells derived from plants or mammals may be used. The host cell may be COS7 cell (monkey kidney cells), NSO cell, SP2/0, Chinese hamster ovary (CHO) cell, W138, baby hamster kidney (BHK) cell, MDCK, myeloma cell line, HuT 78 cell, or 293 cell, but is not limited thereto.

Transformation and/or transfection into the host cell may be performed using any method of introducing a nucleic acid into an organism, cell, tissue, or organ, and may be performed by selecting an appropriate standard technique depending on the host cell, as known in the art. Specifically, electroporation, protoplast fusion, calcium phosphate (CaPO₄) precipitation, calcium chloride (CaCl₂) precipitation, stirring using silicon carbide fiber, agrobacteria-mediated transformation, polyethylene glycol (PEG), dextran sulfate, lipofectamine, a drying/inhibition-mediated transformation method, or the like may be performed, but the present invention is not limited thereto.

A method for producing a humanized antibody of the present invention or an antigen-binding fragment thereof includes culturing the host cell.

The method for producing a humanized antibody or antigen-binding fragment thereof may further include expressing the humanized antibody or antigen-binding fragment thereof in the host cell.

The culturing of the host cell may be performed according to an appropriate medium and culture conditions known in the art. This culture process may be easily adjusted and used by those skilled in the art depending on the selected strain. Cell culture is divided into suspension culture and adherent culture depending on the cell growth method, and batch culture, fed-batch culture, and continuous culture depending on the culture method. The medium used for culture should appropriately meet the requirements of the specific strain.

In animal cell culture, the medium contains various carbon sources, nitrogen sources, and trace element components. Examples of the carbon sources that may be used include carbohydrates such as glucose, sucrose, lactose, fructose, maltose, starch, and cellulose, fats such as soybean oil, sunflower oil, castor oil, and coconut oil, fatty acids such as palmitic acid, stearic acid, and linoleic acid, alcohols such as glycerol and ethanol, and organic acids such as acetic acid, and these carbon sources may be used alone or in combination.

Examples of the nitrogen sources include organic nitrogen sources such as peptone, yeast extract, broth, malt extract, corn steep liquor (CSL), and soybean meal, and inorganic nitrogen sources such as urea, ammonium sulfate, ammonium chloride, ammonium phosphate, ammonium carbonate, and ammonium nitrate, and these nitrogen sources may be used alone or in combination.

The medium may contain potassium dihydrogen phosphate, dipotassium hydrogen phosphate, and a corresponding sodium-containing salt as a phosphorous source. In addition, the medium may contain a metal salt such as magnesium sulfate or iron sulfate. In addition, an amino acid, a vitamin, an appropriate precursor, and the like may be contained.

In the culturing step, the pH of the culture may be adjusted by adding compounds such as ammonium hydroxide, potassium hydroxide, ammonia, phosphoric acid, and sulfuric acid to the culture in an appropriate manner. In addition, during culturing, foam generation may be suppressed by using an anti-foaming agent such as fatty acid polyglycol ester. In addition, in order to maintain the aerobic state of the culture, oxygen or oxygen-containing gas (for example, air) is injected into the culture. The temperature of the culture may be usually a temperature of 20°C to 45°C or 25°C to 40°C.

The production method may further include recovering the humanized antibody or antigen-binding fragment thereof expressed in the host cell. The humanized antibody or antigen-binding fragment thereof obtained by culturing the transformed host cell may be used in an unpurified state, or may be further purified to high purity using various conventional methods such as dialysis, salt precipitation, and chromatography. When using chromatography, the type and order of columns may be selected from ion exchange chromatography, size exclusion chromatography, affinity chromatography, and the like depending on the characteristics of the antibody, culture method, and the like.

### 3. Use of humanized antibody of the present invention for detecting TM4SF4

Still another aspect of the present invention provides a use of the humanized antibody or antigen-binding fragment thereof for detecting TM4SF4. Specifically, the present invention provides a composition for detecting TM4SF4, a kit for detecting TM4SF4, and a method for detecting TM4SF4.

Since the descriptions of the humanized antibody, antigen-binding fragment thereof, TM4SF4, and the like are the same as those described in "1. *Humanized antibody or antigen-binding fragment thereof that specifically binds to TM4SF4",* the descriptions are omitted to avoid repetitive descriptions.

The composition for detecting TM4SF4 of the present invention contains the humanized antibody or antigen-binding fragment thereof, and the kit for detecting TM4SF4 of the present invention contains the composition for detecting TM4SF4.

In addition, the method for detecting TM4SF4 of the present invention includes bringing the humanized antibody or antigen-binding fragment thereof into contact with a sample to be detected that is expected to contain TM4SF4.

The composition for detecting TM4SF4 and the kit containing the same may effectively detect TM4SF4 by bringing the humanized antibody or antigen-binding fragment thereof that specifically binds to TM4SF4 into contact with a sample to be detected to form an antigen-antibody complex.

The antigen-antibody complex refers to a combination of TM4SF4 and an antibody that recognizes TM4SF4 to identify tumor or cancer cells expressing TM4SF4 in a sample.

A quantification method of TM4SF4 antigen using the composition for detecting TM4SF4, the kit containing the same, or the humanized antibody or antigen-binding fragment thereof may be performed by confirming the formation of an antigen-antibody complex, and the confirmation of the formation of the antigen-antibody complex may be performed by enzyme-linked immunosorbent assay (ELISA), Western blotting, immunofluorescence, immunohistochemistry staining, flow cytometry, immunocytochemistry, radioimmunoassay (RIA), immunoprecipitation assay, immunodiffusion assay, complement fixation assay, protein chip, and the like, but is not limited thereto. The enzyme-linked immunosorbent assay (ELISA) includes various ELISA methods, for example, Direct ELISA using a labeled antibody that recognizes an antigen attached to a solid support, Indirect ELISA using a labeled secondary antibody that recognizes a captured antibody in a complex of an antibody that recognizes an antigen attached to a solid support, Direct sandwich ELISA using another labeled antibody that recognizes an antigen in an antigen-antibody complex attached to a solid support, and Indirect sandwich ELISA in which another labeled antibody that recognizes an antigen in an antigen-antibody complex attached to a solid support is reacted, and then a labeled secondary antibody that recognizes the other labeled antibody is used.

Examples of a label that enables the formation of an antigen-antibody complex to be measured qualitatively or quantitatively include, but are not limited to, an enzyme, a fluorescent substance, a ligand, a luminescent substance, a microparticle, a redox molecule, and a radioisotope. Examples of the enzyme include, but are not limited to, β-glucuronidase, β-D-glucosidase, β-D-galactosidase, urease, peroxidase, alkaline phosphatase, acetylcholinesterase, glucose oxidase, hexokinase and GDPase, RNase, glucose oxidase and luciferase, phosphofructokinase, phosphoenolpyruvate carboxylase, aspartate aminotransferase, phosphoenolpyruvate decarboxylase, and β-lactamase.

### 4. Use of humanized antibody of the present invention for preventing or treating cancer, for inhibiting cancer stem cell growth, and for assisting radiation anticancer treatment

Still another aspect of the present invention provides a pharmaceutical composition for preventing or treating cancer, a composition for inhibiting cancer stem cell growth, and a composition for assisting radiation anticancer treatment, containing the humanized antibody or antigen-binding fragment thereof.

Since the descriptions of the humanized antibody, antigen-binding fragment thereof, TM4SF4, and the like are the same as those described in "1. *Humanized antibody or antigen-binding fragment thereof that specifically binds to TM4SF4",* the descriptions are omitted to avoid repetitive descriptions.

However, the humanized antibody of the present invention or the antigen-binding fragment thereof may have a CDR sequence that is identical to or has a modified portion of a CDR sequence of ECL-2B7 antibody, and for the characteristics and effects of the ECL-2B7 antibody, Korean Patent Laid-Open Publication No. 2021-0071856 may be referred to.

The pharmaceutical composition for preventing or treating cancer of the present invention contains the humanized antibody or antigen-binding fragment thereof.

The humanized antibody or antigen-binding fragment thereof may bind to TM4SF4 with high affinity, and the TM4SF4 is known to be overexpressed on a surface of a cancer cell. Accordingly, the humanized antibody or antigen-binding fragment thereof may be used to target cancer cells.

The humanized antibody or antigen-binding fragment thereof in the composition may be used alone or in combination with a conventional pharmaceutically acceptable carrier for treatment, prevention, and diagnosis of a hyperproliferative disease such as cancer.

The cancer may be, for example, lung cancer, stomach cancer, colon cancer, colorectal cancer, triple-negative breast cancer, glioblastoma, head and neck cancer, breast cancer, ovarian cancer, kidney cancer, bladder cancer, prostate cancer, endometrial cancer, salivary gland cancer, or thyroid cancer, more specifically, may be lung cancer, breast cancer, liver cancer, kidney cancer, stomach cancer, pancreatic cancer, or brain cancer, and in particular, may be non-small cell lung cancer or pancreatic cancer, but is not limited thereto.

In the present invention, the cancer may be cancer caused by overexpression, amplification, mutation, or activation of TM4SF4, and may particularly refer to cancer in which TM4SF4 is overexpressed.

The composition may be in the form of a pharmaceutical composition, a quasi-drug composition, or a health food composition.

The composition for preventing or treating cancer of the present invention may further contain a pharmaceutically acceptable carrier. The "pharmaceutically acceptable" means that a substance does not inhibit activity of an active ingredient and does not have toxicity beyond what an application (prescription) target is adaptable to, and the "carrier" is defined as a compound that facilitates addition of a compound into cells or tissue.

The pharmaceutical composition of the present invention may be administered alone or in combination with any convenient carrier and the like, and a formulation to be administered may be a single dosage or repeated dosage form. The pharmaceutical composition may be a solid formulation or a liquid formulation. The solid formulation includes, but is not limited to, a powder, a granule, a tablet, a capsule, a suppository, and the like. The solid formulation may include, but is not limited to, a carrier, a flavoring agent, a binder, a preservative, a disintegrant, a lubricant, a filler, and the like. The liquid formulation includes water, a solution such as a propylene glycol solution, a suspension, an emulsion, and the like, but is not limited thereto, and may be prepared by adding an appropriate colorant, flavoring agent, stabilizer, thickener, and the like. For example, a powder may be prepared by simply mixing a tri-hydroxy derivative of a polyunsaturated fatty acid, which is an active ingredient of the present invention, with an appropriate pharmaceutically acceptable carrier such as lactose, starch, or microcrystalline cellulose. A granule may be prepared by mixing the tri-hydroxy derivative of the polyunsaturated fatty acid of the present invention, an appropriate pharmaceutically acceptable carrier, and an appropriate pharmaceutically acceptable binder such as polyvinylpyrrolidone or hydroxypropyl cellulose, and then utilizing a wet granulation method using a solvent such as water, ethanol, or isopropanol, or a dry granulation method using a compressive force. In addition, a tablet may be prepared by mixing the granule with an appropriate pharmaceutically acceptable lubricant such as magnesium stearate, and then tableting the mixture using a tableting machine.

The pharmaceutical composition may be administered in the form of an oral agent, an injection (for example, an intramuscular injection, an intraperitoneal injection, an intravenous injection, an infusion, a subcutaneous injection, or an implant), an inhalation agent, a nasal administration agent, a vaginal agent, a rectal administration agent, a sublingual agent, a transdermal agent, a topical agent, or the like, depending on a disease to be treated and a condition of an individual, but is not limited thereto. Depending on a route of administration, the pharmaceutical composition may be formulated into an appropriate dosage unit formulation containing a pharmaceutically acceptable carrier, additive, and vehicle, which are commonly used and non-toxic.

The pharmaceutical composition may be administered at a daily dosage of about 0.0001 mg/kg to about 10 g/kg, and may be administered at a daily dosage of about 0.001 mg/kg to about 1 g/kg. However, the dosage may vary depending on a degree of purification of the mixture, a patient's condition (age, gender, weight, or the like), and severity of the condition being treated. If necessary, for convenience, the total daily dosage may be administered in divided doses several times throughout a day.

The composition for inhibiting cancer stem cell growth of the present invention contains the humanized antibody or antigen-binding fragment thereof.

The cancer stem cell (CSC) refers to an undifferentiated cell having the ability to differentiate into various cancer cells. Cancer stem cells exist in malignant tumor tissues in an amount of about 1 to 2% and have self-replication ability and pluripotency, which are characteristics of normal stem cells, but have abnormalities in self-regulatory function, which causes cell division to be activated, leading to an increase in cell number and differentiation into malignant tumor cells. Due to these characteristics of cancer stem cells, general cancer cells are removed through anticancer treatment, but cancer stem cells survive, and it is known that cancer recurrence and metastasis occur due to some of the surviving cancer stem cells.

Specifically, the cancer stem cell of the present invention may be a cancer cell that overexpresses aldehyde dehydrogenase 1 (ALDH1) protein, which is one of the markers of cancer stem cells, or has positive protein activity.

The humanized antibody of the present invention or the antigen-binding fragment thereof may selectively inhibit cancer stem cells, and in particular, may obtain excellent anticancer effects by killing a cancer cell population including cancer stem cells that are highly resistant to anticancer treatment. The humanized antibody or antigen-binding fragment thereof may inhibit the growth of cancer stem cells by reducing the self-renewal ability, invasion ability, and migration ability of cancer stem cells.

In a specific embodiment of the present invention, adenocarcinoma cell line A549 among lung cancer cells, and MIAPaCa-2 cells, which are a pancreatic cancer cell line, were treated with the humanized antibody of the present invention, and then, the expression level of ALDH1, which is a cancer stemness marker, was measured. As a result, it was confirmed that the humanized antibody of the present invention inhibits the stem cell transformation of cancer cells, as the expression level of ALDH1 protein was significantly reduced.

The antibody or antigen-binding fragment thereof may be used to prevent or treat cancer having cancer stem cell characteristics, but is not limited thereto. The cancer having cancer stem cell characteristics has resistance to the existing anticancer treatment and has a poor prognosis; thus, treatment different from the existing anticancer treatment should be applied thereto. For example, even in patients with the same type of cancer, in a case where the cancer has a high proportion of cancer stem cells, the patient will not be able to obtain cancer treatment effects from the existing known cancer treatment such as anticancer drug administration or radiation therapy. Therefore, even if it is the same type of cancer, in a case where a proportion of cancer stem cells is high in cells of a cancer lesion site, it is significantly important to apply a novel treatment method that is different from the existing anticancer treatment.

The cancer having cancer stem cell characteristics described above may be cancer having a high proportion of cancer stem cells in a cell population that constitutes the cancer. Considering that the proportion of cancer stem cells among general cancer cells is about 1% or more and less than 5%, for example, cancer may be defined as having cancer stem cell characteristics when a proportion of cancer stem cells in a cell population that constitutes cancer is 5% or more, 10% or more, 30% or more, 50% or more, or 70% or more, and as described above, the cancer may be characterized by resistance to the existing anticancer treatment and a poor prognosis for anticancer treatment. Specifically, in the present invention, the cancer having cancer stem cell characteristics may be cancer that overexpresses ALDH1. The cancer that overexpresses ALDH1 may be cancer in which a proportion of cancer stem cells that express ALDH1 or whose activity is positive is relatively higher than that of general cancer.

Specifically, the cancer that overexpresses ALDH1 may be at least one selected from the group consisting of lung cancer, breast cancer, liver cancer, kidney cancer, stomach cancer, pancreatic cancer, and brain cancer, but is not limited thereto.

The prevention or treatment of cancer may be intended to prevent or treat cancer chemoresistance, cancer recurrence, or cancer metastasis during cancer treatment or after cancer treatment, by reducing the regenerative ability, growth ability, invasion ability, or migration ability of cancer stem cells.

In a specific embodiment of the present invention, adenocarcinoma cell line A549 among lung cancer cells, and MIAPaCa-2 cells, which are a pancreatic cancer cell line, were treated with the humanized antibody of the present invention, and then, the migration and invasion of cells were observed through a transwell. As a result, it was shown that the migration and invasion of cancer cells were reduced by about 40%, confirming that the humanized antibody of the present invention plays an important role in inhibiting the metastasis of cancer cells.

The composition for assisting radiation anticancer treatment of the present invention contains the humanized antibody or antigen-binding fragment thereof.

The composition contains the humanized antibody or antigen-binding fragment thereof as an effective ingredient for reducing radiation resistance of cancer-related cells.

The cancer-related cells are cells that constitute cancer, and may have characteristics such as an irregular shape compared to normal cells, indefinite proliferation, and weak bonding with surrounding cells. Specifically, the cancer-related cell may be a cancer cell or a cancer stem cell, and specifically, may be a cancer stem cell.

The cancer stem cells may be undifferentiated cells having the ability to differentiate into various cancer cells, and specifically, may be cancer cells that express ALDH1 or have positive activity for ALDH1. In the present invention, the cancer stem cells may have the characteristics of not being inhibited in cell proliferation by radiation exposure, not being reduced in self-renewal ability, and not being inhibited in migration and invasion ability.

In addition, the cancer-related cells may be highly resistant to radiation, that is, highly resistant to radiation therapy, and may be substantially highly resistant to radiation, making anticancer treatment using radiation exposure impossible.

The anticancer may be intended to inhibit proliferation of cancer-related cells, inhibit metastasis and invasion, and induce apoptosis through radiation exposure, surgical operation, and chemotherapy.

In the present invention, in the anticancer, the humanized antibody or antigen-binding fragment thereof may be administered in combination with radiation exposure. As such, when the humanized antibody or antigen-binding fragment is administered in combination with radiation exposure, the radiation resistance of cancer-related cells is suppressed by the antibody or antigen-binding fragment, such that the anticancer treatment effect may be maximized by radiation exposure, and the recurrence and metastasis of cancer may be further prevented.

In a specific embodiment of the present invention, adenocarcinoma cell line A549 among lung cancer cells, and MIAPaCa-2 cells, which are a pancreatic cancer cell line, were treated with the humanized antibody of the present invention, and then, the radiation resistance of cancer cells was measured by analyzing colony formation. As a result, when the humanized antibody of the present invention was treated, it was confirmed that the number of colonies decreased after radiation exposure, resulting in inhibition of cell growth. Therefore, it was confirmed that the humanized antibody of the present invention has the effect of reducing the resistance of cancer cells to radiation.

### 5. Use of humanized antibody of the present invention for assisting anticancer immunotherapy

Still another aspect of the present invention provides a composition for assisting anticancer immunotherapy containing the humanized antibody or antigen-binding fragment thereof.

Since the descriptions of the humanized antibody, antigen-binding fragment thereof, TM4SF4, and the like are the same as those described in "*1*. *Humanized antibody or antigen-binding fragment thereof that specifically binds to TM4SF4",* the descriptions are omitted to avoid repetitive descriptions.

Similarly, since the descriptions of the composition, anticancer, and the like are the same as those described in "*4. Use of humanized antibody of the present invention for preventing or treating cancer, for inhibiting cancer stem cell growth, and for assisting radiation anticancer treatment",* the descriptions are omitted to avoid repetitive descriptions.

The anticancer immunotherapy assistance means that it may promote or assist the elimination of cancer cells by immune cells by strengthening the immune system of a cancer patient and suppressing the immune evasion mechanism of cancer cells, and specifically, it may enhance immune activity by enhancing antibody-dependent cellular cytotoxicity (ADCC), inhibiting the expression of programmed death-ligand (PD-L1), or inhibiting the expression of B7H4.

The ADCC refers to a process by which immune cells lyse target cells bound to a specific antibody on their cell surfaces, and requires immune cells known as natural killer (NK) cells that bind to an antibody. When the humanized antibody of the present invention or the antigen-binding fragment thereof is treated, the humanized antibody or antigen-binding fragment thereof binds to cancer cells, and the antibody-dependent cellular cytotoxicity function of NK cells may be enhanced due to the binding.

In a specific embodiment of the present invention, the humanized antibody of the present invention was treated to hepatocytes as a control, and A549 cells, which are an adenocarcinoma cell line among lung cancer cells, and MIAPaCa-2 cells, which are a pancreatic cancer cell line, and then the antibody-dependent cellular cytotoxicity (ADCC) function was confirmed. As a result, it was confirmed that no ADCC was observed in the control, whereas a clear ADCC was observed in the cancer cells.

The PD-L1 refers to a ligand of an immune checkpoint receptor of cancer cells, which causes an immune evasion mechanism of cancer cells for immune cells, thereby preventing the elimination of cancer cells by immune cells. When the humanized antibody of the present invention or the antigen-binding fragment thereof is treated, the expression of the PD-L1 gene or protein may be inhibited.

The B7-H4 is a negative regulator of T cell function, and ligation of T cells may inhibit its growth, cytokine secretion, and cytotoxicity. B7-H4 is known to be overexpressed in various solid tumors, and the expression of B7-H4 in tumor is known to be correlated with poor prognosis. In particular, B7-H4 directly inhibits T cell activity, which causes an immune evasion mechanism of cancer cells for immune cells, thereby preventing the elimination of cancer cells by immune cells. When the humanized antibody of the present invention or the antigen-binding fragment thereof is treated, the expression of the B7-H4 gene or protein may be inhibited.

In a specific embodiment of the present invention, A549 cells, which are an adenocarcinoma cell line among lung cancer cells, and MIAPaCa-2 cells, which are a pancreatic cancer cell line, were treated with the humanized antibody of the present invention, and then, changes in the expression levels of PD-L1 and B7-H4 genes and proteins were measured. As a result, it was confirmed that the expression of PD-L1 and B7-H4 genes and proteins was significantly reduced.

Therefore, the humanized antibody of the present invention and the antigen-binding fragment thereof have the effect of enhancing the ADCC effect of immune cells and inhibiting the expression of PD-L1 and B7-H4, thereby strengthening the immune system of a cancer patient and promoting the elimination of cancer cells by immune cells by inhibiting the immune evasion mechanism of cancer cells.

Hereinafter, the present invention will be described in detail with reference to examples.

However, the following examples are intended to illustrate the present invention specifically, and the present invention is not limited by the following examples.

### [Example 1]

### Confirmation of cell growth according to treatment with anti-TM4SF4 humanized antibody in lung cancer cells and pancreatic cancer cells

It was confirmed whether the humanized antibody of the present invention reduces the growth of the cancer cells in A549, which is an adenocarcinoma cell line among lung cancer cells, and MIAPaCa-2, which is a pancreatic cancer cell line.

First, a protease inhibitor cocktail (Sigma-Aldrich, St Louis, UAS) was mixed with TX100 lysis buffer [20 mM Tris-HCl (pH 7.5) buffer containing 150 mM NaCl, 1 mM EGTA, 1 mM EDTA, and 0.5% Triton X-100], and the cancer cells were treated with the mixed solution.

A protein concentration was measured by Western blotting using Bradford reagent (Bio-Rad, Hercules, CA, USA). For Western blotting assay, equal amounts of proteins were separated on a 8 to 15% sodium dodecyl sulfate (SDS)-polyacrylamide membrane, and the separated proteins were transferred to Hybond nitrocellulose membrane (Amersham Pharmacia, Pittsburgh, PA, USA). The membrane was blocked in phosphate-buffered saline (PBS) buffer containing nonfat milk (10%) and Tween 20 (0.1%) at room temperature for 1 hour. In the membrane, TM4SF4 and β-actin were treated with antibodies overnight in a low-temperature chamber. Thereafter, the membrane was washed with Tris-buffered saline, the membrane was treated with a peroxidase-labeled secondary antibody (Abcam), and then, the protein was visualized using Westzol enhanced chemiluminescence detection kit (iNtRON Biotechnolgy).

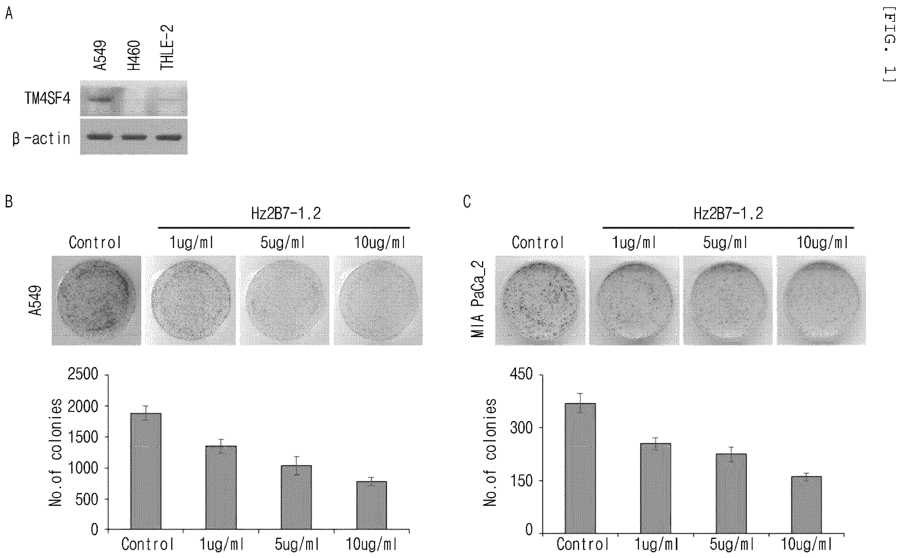

Western blotting was performed for TM4SF4 protein in A549, H460, and THLE-2 cells using the same method as above. As a result, as illustrated in FIG. 1A, it was confirmed that TM4SF4 was expressed in A549 cancer cells, but was not expressed in H460 and THLE-2 cells (FIG. 1A).

2 x 10³ cells of A549 and 2 x 10³ cells of MIAPaCa-2 expressing TM4SF4 were cultured in a 60 mm cell culture dish and treated with anti-TM4SF4 humanized antibodies of the present invention (Hz-2B7-1.2) at concentrations of 1 µg/ml, 5 µg/ml, and 10 µg/ml, respectively. Five days after treatment, the A549 and MIAPaCa-2 cells were stained with a 0.5% (w/v) crystal violet solution and observed to count the number of colonies, and a colony survival rate was expressed as a percentage (%) relative to the control. IgG antibodies were used as the antibodies treated to the control.

As a result, in the A549 and MIAPaCa-2 cells, the number of stained colonies decreased in proportion to the concentration of antibodies treated compared to the control (FIGS. 1B and 1C). Therefore, it was confirmed that TM4SF4, which is a membrane protein, plays an important role in the growth of the adenocarcinoma cell line among non-small cell lung cancers and pancreatic cancer cell line, and the growth ability of cells was significantly reduced when the TM4SF4 was bound to an anti-TM4SF4 humanized antibody.

### [Example 2]

### Confirmation of radiation resistance in lung cancer cells and pancreatic cancer cells

In order to confirm whether radiation resistance is reduced through cell growth when the adenocarcinoma cell line A549, and the MIAPaCa-2 cells, which are a pancreatic cancer cell line, were treated with the humanized antibody of the present invention, the cancer cell lines were treated with the humanized antibody of the present invention (Hz-2B7-1.2), and then, a colony staining experiment was performed using the same method as described in Example 1. At this time, the treatment with the humanized antibody was performed before radiation (3 Gy) exposure.

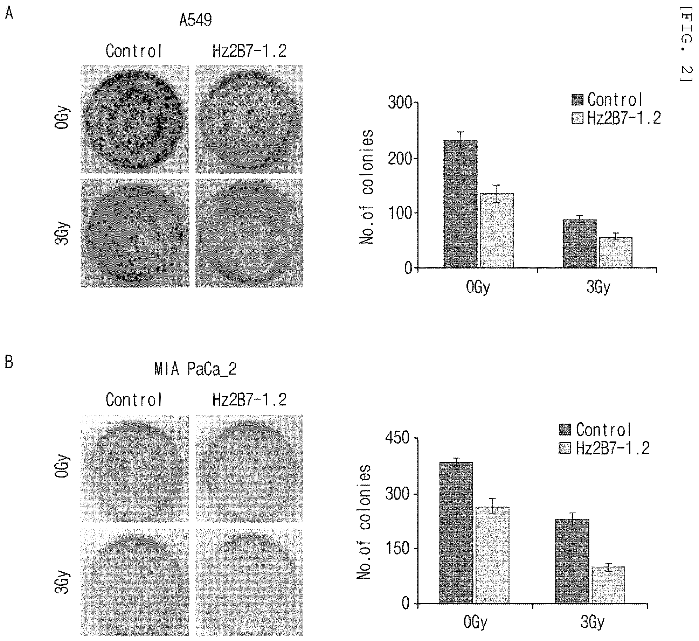

As a result, when the A549 cells, which are an adenocarcinoma cell line among the non-small cell lung cancers, and MIAPaCa-2 cells, which are a pancreatic cancer cell line, were treated with the humanized antibody of the present invention, both the number of cultured colonies and the number of cultured colonies after radiation exposure decreased, confirming that cell growth was reduced (FIG. 2). Therefore, it may be confirmed that when the function of TM4SF4 is inhibited by treating the A549 and MIAPaCa-2 cells expressing TM4SF4 with the humanized antibody of the present invention, the radiation resistance is reduced.

### [Example 3]

### Confirmation of cell migration through wound healing in lung cancer cells and pancreatic cancer cells

The present experiment was conducted to determine whether the treatment with the humanized antibody of the present invention affects not only cell growth but also cell migration ability. The A549 and MiaPaCa-2 cells were treated with the humanized antibody of the present invention (Hz-2B7-1.2), and after 72 hours, the A549 and MiaPaCa-2 cells were transferred to a 35-mm culture dish, cultured until the confluency was 90% or higher, and thoroughly washed with PBS, the culture medium was changed to RPMI 1640 medium containing 0.5% FBS, and the cells were cultured overnight at 5% CO₂ and 37°C to induce functional inhibition of TM4SF4. The bottom of the culture dish was wounded by drawing two lines using a 200 µl tip to scrape the cells, the culture dish was slowly washed again with PBS and filled with cell culture medium again, and the cells were cultured and observed after 36 hours. A change in spacing between the wounds was expressed as a relative wound area.

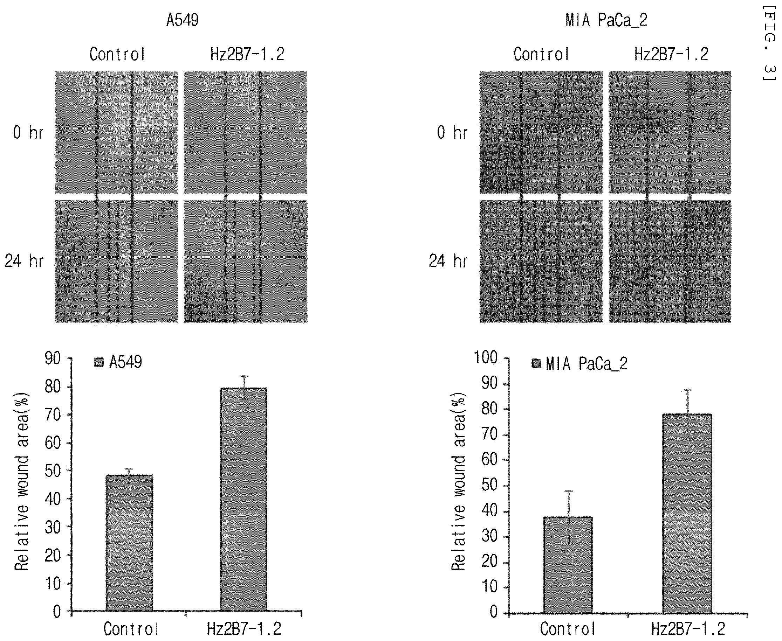

As a result, as illustrated in FIG. 3, it was confirmed that the migration of the A549 and MiaPaCa-2 cells treated with the humanized antibody of the present invention was significantly reduced compared to the control in which the relative wound area was lowered to 50% or less due to increased cell migration for up to 24 hours. Therefore, it was confirmed that continuous cell migration was inhibited by treating the adenocarcinoma cell line A549 and the pancreatic cancer cell line MiaPaCa-2 with the humanized antibody of the present invention.

### [Example 4]

### Confirmation of cell migration and invasion through transwell in lung cancer cells and pancreatic cancer cells

Using a transwell, changes in cell migration and cell invasion ability were confirmed according to the treatment with the humanized antibody of the present invention.

### 4-1. Confirmation of reduced cell migration in treatment with humanized antibody of the present invention in A549 and MIAPaCa-2 cells

In order to observe cell migration, A549 and MIAPaCa-2 cells were treated with the humanized antibody of the present invention (Hz-2B7-1.2) in the same manner as described in Example 3 to induce functional inhibition of TM4SF4. After 72 hours, the A549 and MIAPaCa-2 cells were cultured in RPMI 1640 medium containing 10% fetal bovine serum (FBS, Hyclone) and antibiotics (100 U/ml penicillin and 100 µg/ml streptomycin, Hyclone) and introduced into a chamber of a transwell (Cell biolabs) at 2 × 10⁵/300 µl. 500 µl of RPMI 1640 medium without fetal bovine serum was added to the lower chamber, and culture was performed at 5% CO₂ and 37°C for 24 hours. After the culture was completed, the cells which were migrated to the lower surface of the transwell were stained with CyQuant^{™} GR staining reagent, and the number of stained cells was observed.

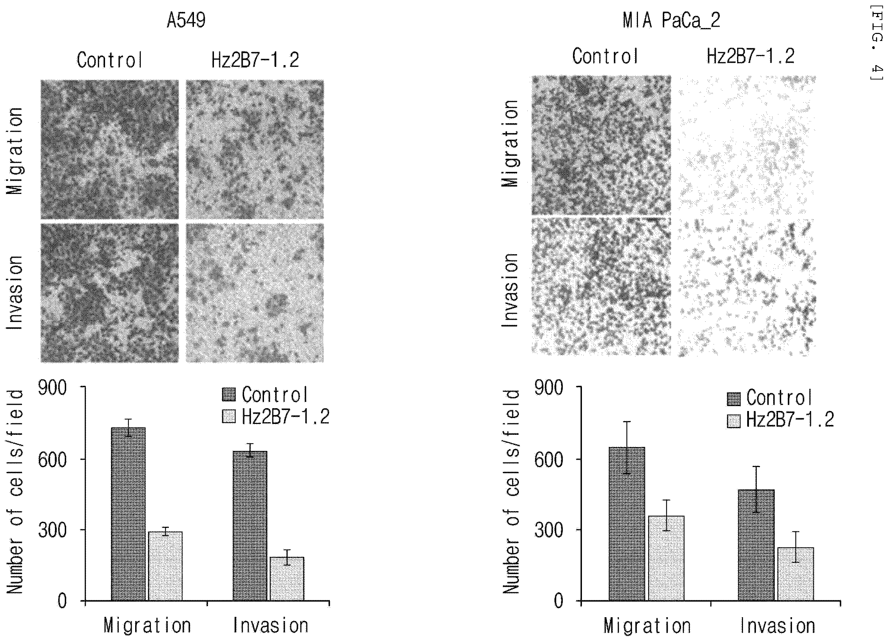

As a result, as illustrated in FIG. 4, it was confirmed that the migration of the cells treated with the humanized antibody of the present invention was reduced by about 40% compared to the control in the adenocarcinoma cell line A549 and MIAPaCa-2.

### 4-2. Confirmation of reduced cell invasion in treatment with humanized antibody of the present invention in A549 and MIAPaCa-2 cells

In order to measure cell invasion, A549 and MIAPaCa-2 cells were treated with the humanized antibody of the present invention (Hz-2B7-1.2) in the same manner as described in Example 3 to induce functional inhibition of TM4SF4. After 72 hours, A549 and MIAPaCa-2 cells were prepared at 5 x 10⁵, coated with 10 µl of Matrigel^{™} on the upper surface of the transwell, and dried, and then, the cells were cultured for 30 minutes at room temperature in RPMI 1640 medium without fetal bovine serum. Thereafter, an experiment was performed using a transwell in the same manner as the cell migration measurement method described in Example 4-1. After the culture was completed, the upper surface of the transwell was removed with a cotton swab to observe the invaded cells, the cells were stained with CyQuant^{™} GR staining reagent, and the number of stained cells was observed.

As a result, as can be confirmed in FIG. 4, cell invasion was reduced by about 40% in adenocarcinoma cell line A549 and MIAPaCa-2 cells. Therefore, it was confirmed that the humanized antibody of the present invention has the effect of inhibiting the metastasis of cancer cells by reducing cell migration and invasion.

### [Example 5]

### Confirmation of cell sphere formation in lung cancer cells and pancreatic cancer cells

After treatment with the humanized antibody of the present invention (Hz-2B7-1.2), a sphere forming assay experiment was performed on A549 and MiaPaCa-2 cells. During the sphere forming assay, the A549 and MiaPaCa-2 cells were added to stem cell permissive Dulbecco's Modified Eagle Medium (DMEM-F12; Invitrogen) containing epidermal growth factor (FGF, 20 ng/ml), basic fibroblast growth factor (20 ng/ml), and B27 serum. The cells were dispensed at a density of 1 cell/well or 2 cells/well in ultra-low attachment 96-well plates (Corning, Inc., Corning, NY, USA) and incubated overnight at 37°C in a 5% CO₂ humidified incubator. Thereafter, the presence of single cells in each well was visually confirmed, and spheres were quantified using an inverted phase contrast microscope after 9 to 12 days. Meanwhile, about 2 x 10³ cells were dispensed into a 6-well plate under the culture conditions described above, cultured in a 5% CO₂ humidified incubator, and the number of spheres formed was quantified.

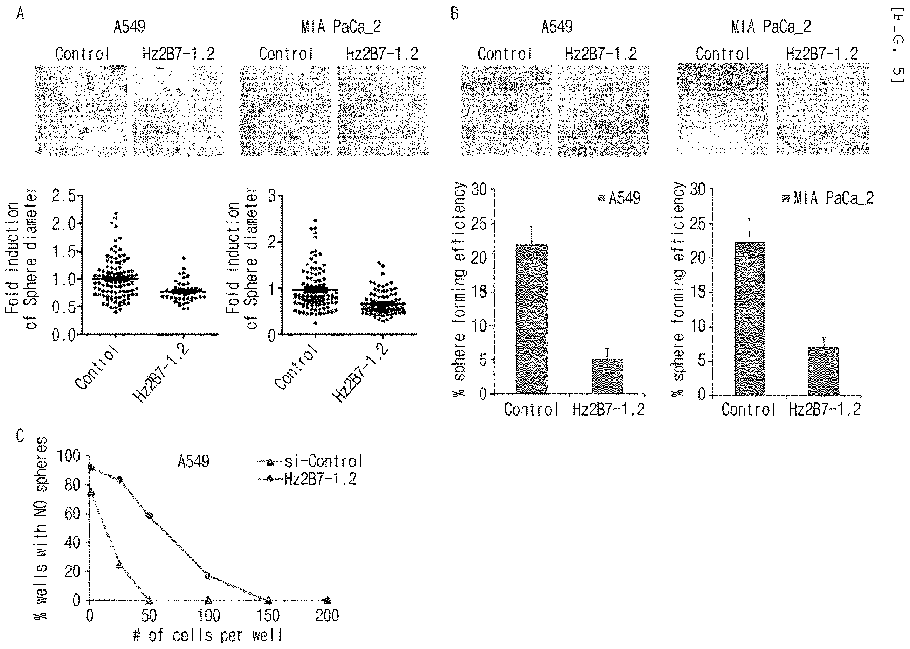

As a result, as illustrated in FIG. 5, it was confirmed that when the humanized antibody of the present invention was treated, sphere formation was significantly inhibited compared to when the IgG antibody was treated as a control. Therefore, it was confirmed that the inhibition of TM4SF4 function by the humanized antibody treatment of the present invention plays an important role in inhibiting cancer cell stemness.

### [Example 6]

### Confirmation of whether epithelial to mesenchymal transition (EMT) of lung cancer cells and pancreatic cancer cells is inhibited

It was investigated whether the humanized antibody of the present invention inhibits the EMT phenomenon. Specifically, the changes in expression levels of EMT markers such as E-cadherin, N-cadherin, Vimentin, Snail, and β-actin proteins were investigated by Western blotting and immunofluorescence when A549 and MIAPaCa-2 cells were treated with the humanized antibody of the present invention (Hz-2B7-1.2).

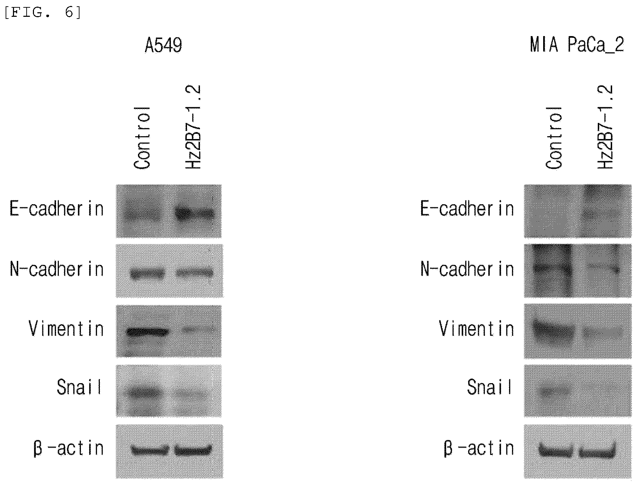

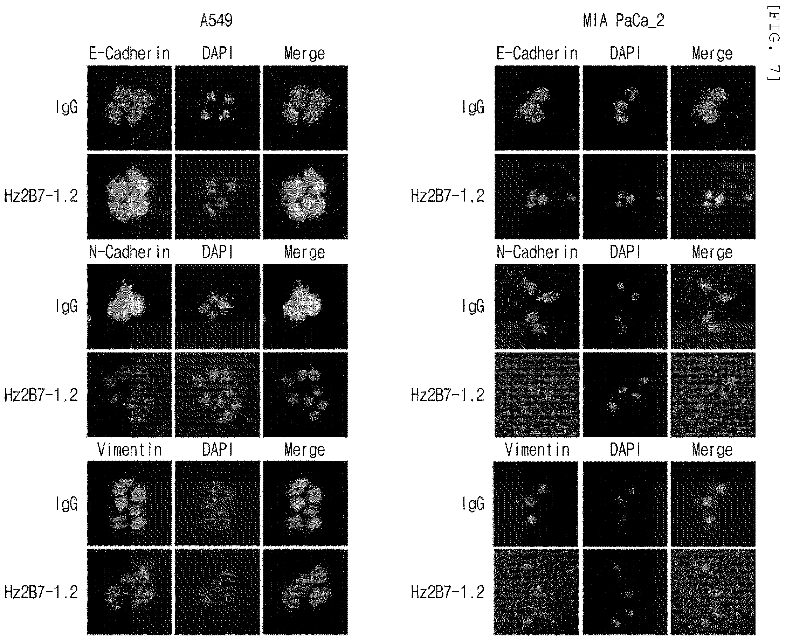

As a result of Western blotting and immunofluorescence experiments, it was confirmed that the expression of E-cadherin increased and the expression of N-cadherin and Vimentin decreased according to treatment with the humanized antibody of the present invention (FIGS. 6 and 7). Therefore, this means that the humanized antibody of the present invention effectively inhibits the EMT phenomenon and effectively controls cancer.

### [Example 7]

### Confirmation of expression of ALDH1 and CD44 in lung cancer cells and pancreatic cancer cells

In order to confirm whether the expression of ALDH1 and CD44, which are representative markers of cancer cell stemness, is reduced by the humanized antibody of the present invention (Hz-2B7-1.2), the change in expression level was investigated through immunofluorescence assay.

In the same manner as in the examples described above, the TM4SF4 humanized antibody was treated to induce functional inhibition of TM4SF4 in A549 and MIAPaCa-2 cells. After 72 hours, the expression level was confirmed by a fluorescene microscope after treatment with an antibody labeled with each antibody fluorescent factor for ALDH1 and CD44.

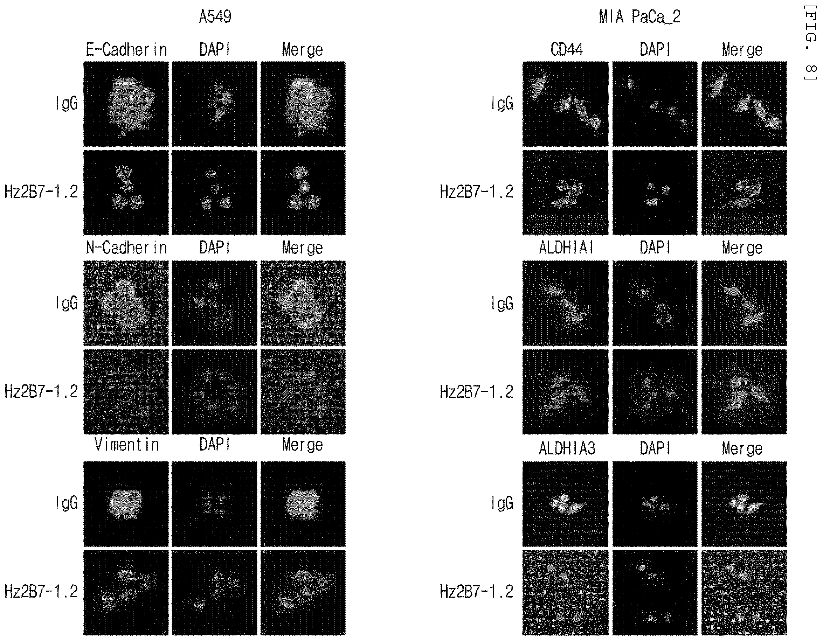

As a result, as illustrated in FIG. 8, it was confirmed that the expression levels of ALDH1 and CD44 within cells were significantly reduced due to inhibition of TM4SF4 function when the humanized antibody of the present invention was treated. That is, it was confirmed that the humanized antibody of the present invention was involved in regulating the expression of ALDH1 and CD44, which are cancer stemness regulation factors within cells.

### [Example 8]

### Confirmation of antibody-dependent cellular cytotoxicity (ADCC) function in lung cancer cells and pancreatic cancer cells

ADCC assay was performed using ADCC Reporter Bioassay, V Variant, and Core Kit (cat. G7010, Promega). A549 cells, MIAPaCa-2 cells, and hepatocyte THLE cells were used as target cells, respectively, and the target cells were inoculated into a white 96-well plate and incubated at 37°C for 20 to 24 hours before assay. The cultured medium was removed from each well, and 25 µl of ADCC Assay Buffer was added the next day. A serially diluted humanized antibody of the present invention (Hz-2B7-1.2) was prepared in a V-bottom 96-well plate and incubated at room temperature. 25 µl of the diluted antibody was inoculated into a white 96-well plate. As ADCC bioassay effector cells (CPM), cryopreserved cells that may be thawed, proliferated, and stored for long-term use were used as effector cells, and 25 µl of the effector cells were added to the target cells (effector:target ratio was 12:1) and incubated at 37°C for 6 hours. After 6 hours, the assay plate was incubated at ambient temperature (20 to 22°C) for 15 minutes. Thereafter, luciferase assay was performed to visualize cytotoxicity. 75 µl of Bio-Glo^{™} Luciferase Assay Reagent was suspended in each sample (well) and incubated at ambient temperature for 5 to 30 minutes. Luciferase activity was measured using ONE-Glo Luciferase Assay Reagent (Promega) and GLOMAX^{™} 96 MICROPLATE LUMINOMETER (Promega). The luciferase activity was normalized with anti-IgG.

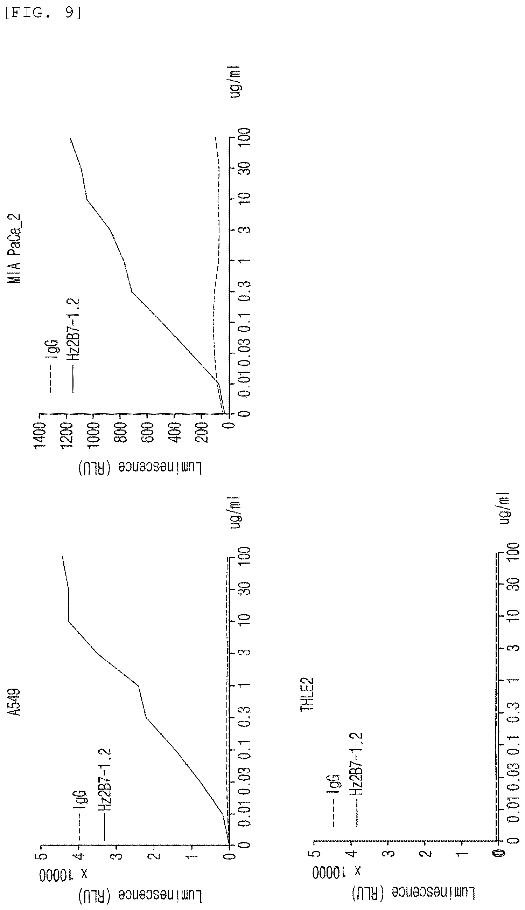

As a result, as illustrated in FIG. 9, antibody-dependent cellular cytotoxicity was clearly observed in the A549 and MIAPaCa-2 cells, whereas no antibody-dependent cellular cytotoxicity was observed in the hepatocyte THLE cells used as a control. Therefore, it was confirmed that the humanized antibody of the present invention does not act on normal hepatocytes but specifically acts on cancer cells, thereby enhancing cytotoxicity.

### [Example 9]

### Confirmation of expression of programmed death-ligand 1 (PD-L1) and B7H4 in lung cancer cells and pancreatic cancer cells

A549 cells, which are an adenocarcinoma cell line, and MIAPaCa-2 cells, which are a pancreatic cancer cell line, were treated with the humanized antibody of the present invention (Hz-2B7-1.2), and then, the changes in the protein levels of PD-L1 and B7H4, which are ligands of immune checkpoint receptors present in cancer cells for the purpose of immune evasion, were confirmed using Western blotting in the same manner as in Example 7. In addition, the changes in gene expression of PD-L1 and B7H4 were confirmed using RT-PCR method.

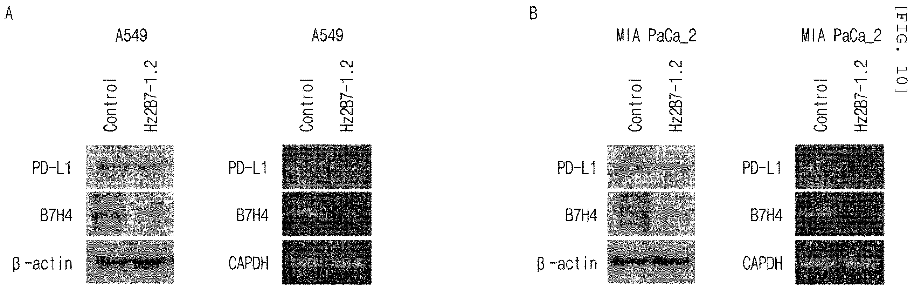

As a result, as illustrated in FIG. 10, it was confirmed that the expression of PD-L1 and B7H4 proteins and genes was significantly reduced in the A549 and MIAPaCa-2 cells according to the treatment with the humanized antibody of the present invention. This means that the humanized antibody of the present invention functions as an immune anticancer antibody.

## Claims

1. A humanized antibody or antigen-binding fragment thereof, comprising:
a heavy chain variable region containing FR-H1 having an amino acid sequence of SEQ ID NO: 1, FR-H2 having an amino acid sequence of SEQ ID NO: 2, FR-H3 having an amino acid sequence of SEQ ID NO: 3, and FR-H4 having an amino acid sequence of SEQ ID NO: 4; and
a light chain variable region containing FR-L1 having an amino acid sequence of SEQ ID NO: 5, FR-L2 having an amino acid sequence of SEQ ID NO: 6, FR-L3 having an amino acid sequence of SEQ ID NO: 7, and FR-L4 having an amino acid sequence of SEQ ID NO: 8,
wherein the humanized antibody or antigen-binding fragment thereof specifically binds to TransMembrane 4 Superfamily Member 4 (TM4SF4).

2. The humanized antibody or antigen-binding fragment thereof of claim 1, wherein the heavy chain variable region further contains at least one CDR selected from the group consisting of CDR-H1 having an amino acid sequence of SEQ ID NO: 9, CDR-H2 having an amino acid sequence of SEQ ID NO: 10, and CDR-H3 having an amino acid sequence of SEQ ID NO: 11, and
the light chain variable region further contains at least one CDR selected from the group consisting of CDR-L1 having an amino acid sequence of SEQ ID NO: 12 or SEQ ID NO: 13, CDR-L2 having an amino acid sequence of SEQ ID NO: 14, and CDR-L3 having an amino acid sequence of SEQ ID NO: 15.

3. The humanized antibody or antigen-binding fragment thereof of claim 1, wherein the heavy chain variable region has an amino acid sequence of SEQ ID NO: 16, and
the light chain variable region has an amino acid sequence of SEQ ID NO: 17 or SEQ ID NO: 18.

4. The humanized antibody or antigen-binding fragment thereof of claim 1, wherein the humanized antibody or antigen-binding fragment thereof has an amino acid sequence in which the amino acid asparagine is substituted with phenylalanine at position 31 of CDR1 in the light chain variable region.

5. The humanized antibody or antigen-binding fragment thereof of any one of claims 1 to 4, wherein the antigen-binding fragment is any one selected from the group consisting of Fab, F(ab'), F(ab')₂, and Fv.

6. A polynucleotide comprising a base sequence encoding the humanized antibody or antigen-binding fragment thereof of any one of claims 1 to 4.

7. An expression vector comprising the polynucleotide of claim 6.

8. A host cell comprising the expression vector of claim 7.

9. A method for producing a humanized antibody or antigen-binding fragment thereof, the method comprising culturing the host cell of claim 8.

10. A composition for detecting TM4SF4, the composition comprising the humanized antibody or antigen-binding fragment thereof of any one of claims 1 to 4.

11. A kit for detecting TM4SF4, the kit comprising the composition for detecting TM4SF4 of claim 10.

12. A method for detecting TM4SF4, the method comprising bringing the humanized antibody or antigen-binding fragment thereof of any one of claims 1 to 4 into contact with a sample to be detected that is expected to contain TM4SF4.

13. A pharmaceutical composition for preventing or treating cancer, the pharmaceutical composition comprising the humanized antibody or antigen-binding fragment thereof of any one of claims 1 to 4.

14. The pharmaceutical composition of claim 13, wherein the prevention or treatment of cancer is to prevent or treat at least one selected from the group consisting of cancer chemoresistance during cancer treatment, cancer chemoresistance after cancer treatment, cancer recurrence, and cancer metastasis.

15. The pharmaceutical composition of claim 13, wherein the cancer is at least one selected from the group consisting of lung cancer, non-small cell lung cancer, stomach cancer, ovarian cancer, cervical cancer, breast cancer, pancreatic cancer, colon cancer, colorectal cancer, esophageal cancer, skin cancer, thyroid cancer, kidney cancer, liver cancer, head and neck cancer, bladder cancer, prostate cancer, blood cancer, multiple myeloma, acute myeloid leukemia, malignant lymphoma, thymus cancer, osteosarcoma, fibrous tumor, and brain cancer.

16. The pharmaceutical composition of claim 13, wherein the cancer is non-small cell lung cancer or pancreatic cancer.

17. A composition for inhibiting cancer stem cell growth, the composition comprising the humanized antibody or antigen-binding fragment thereof of any one of claims 1 to 4.

18. A composition for assisting radiation anticancer treatment, the composition comprising the humanized antibody or antigen-binding fragment thereof of any one of claims 1 to 4.

19. The composition of claim 18, wherein the humanized antibody or antigen-binding fragment thereof enhances sensitivity of cancer cells, including cancer stem cells, to radiation.

20. A composition for assisting anticancer immunotherapy, the composition comprising the humanized antibody or antigen-binding fragment thereof of any one of claims 1 to 4.

21. The composition of claim 20, wherein the humanized antibody or antigen-binding fragment thereof enhances immune activity by inhibiting the expression of PD-L1 or B7H4.

22. The composition of claim 20, wherein the humanized antibody or antigen-binding fragment thereof enhances antibody-dependent cellular cytotoxicity (ADCC) by reacting with natural killer cells (NK cells).
